# EUROPEAN PATENT SPECIFICATION

(11) **EP 0 515 332 B1**
(45) Date of publication and mention of the grant of the patent: **14.02.1996**
(21) Application number: 92830248.8
(22) Date of filing: 22.05.1992
(51) Int. Cl.: A61M 25/10

(54) **Apparatus provided with sensors for inflating the balloon of catheters used for performing coronary angio-plasty**
Einrichtung mit Sensoren zum Aufblasen des Ballons von Kathetern für die Durchführung einer Koronarangioplastie
Appareil muni de capteurs pour gonfler le ballonnet de cathéters utilisés pour réaliser une angioplastie coronaire

(30) Priority: 24.05.1991 IT BO910180
(43) Date of publication of application: 25.11.1992
(73) Proprietor: MINISTERO DELL' UNIVERSITA' E DELLA RICERCA SCIENTIFICA E TECNOLOGICA, I-00196 Roma (IT)
(72) Inventor: Ottovianelli, Giovanni, 00152 Roma (IT); Trivella, Maria Giovanna, 56100 Pisa (IT); Marraccini, Paolo, 56100 Pisa (IT); Ercolani, Mauro, 00153 Roma (IT); Tomasi, Massimo, 41037 Mirandola - Modena (IT); Menni, Dante, 40128 Bologna (IT)
(74) Representative: Dall'Olio, Giancarlo

(56) References cited:
- US-A- 4 154 227
- US-A- 4 370 982
- US-A- 4 429 724

## Description

The present invention relates to the technical field concerning the manufacturing of instruments and apparatuses for performing coronary angioplasty.

It is known that coronary angioplasty has successfully been performed for long time on patients suffering from obstructing coronary disease.

This therapeutical method consists in enlarging a coronary stenosis, obtained by inflating a balloon catheter inserted through a big peripheral artery.

To this aim a guide catheter, having a large diameter, is made to advance up to the coronary ostium, and then the balloon catheter is inserted through it.

Once located at the stenosis the balloon is inflated for a short time (a few dozens of seconds) to a high pressure.

The inflation, that lasts for a short time, can be repeated several times in a period of many minutes, until a satisfactory enlarging of the stenosis is obtained.

The equipment presently used for performing the coronary angioplasty includes a syringe for injecting the used for inflating the balloon, and provided with suitable connecting means for the balloon catheter.

To reach the required pressure (typically 6-12 bar (atmospheres)) the syringe is equipped with a threaded plunger which can be actuated by means of an esternal hand crank.

On the syringe there are usually mounted a pressure gauge, typically connected to the connecting tubing of the balloon catheter, and a graduated scale printed on the barrel of the syringe for measuring the amount of injected fluid.

However it is awkward to read the values of these parameters and it is not possible to immediately process these parameter, particularly to see as they vary as the method performace progresses.

It is therefore proposed to use a pressure transducer in place of the conventional pressure gauge; the transducer is connected to a proper display.

With this configuration it is possible to reach the aim of facilitating the reading of the values, but it is only allowed to read instantaneous values of pressure and it is not possible to understand and compare the variation of these parameters in the time while the procedure take place.

The US-A-4,651,738 discloses a method for performing percutaneous transluminal angioplasty.
The method involves simultaneous measurement and display of the fluid pressure and volume existing within the balloon catheter as the procedure is performed.

Information is produced which is useful in determining the efficacy of the procedure as it is performed which obviates the needs for arbitrary repeated inflations.

The information is useful also in the subsequent management of the patient's disease.

The publication WO-A-90/11040 describes an electronically controlled syringe system for connection to a balloon catheter and for automatically monitoring, displaying and recording inflation data when the syringe stem is used to inflate the balloon catheter.

The syringe includes a barrel and a plunger and is selectively operable to increase fluid pressure applied to the balloon catheter by sliding the plunger further into the barrel.

The pressure applied can be released by withdrawing the plunger toward the rear of the barrel.

A piezoresistive semiconductor transducer is housed on the barrel of the syringe and senses the positive fluid pressure applied by the syringe.

The electronic signal output by the transducer is input to a controller where the signal is processed so as to derive and record therefrom electronic data representing the magnitude of the applied fluid pressure. The inflation data are shown on a display.

The coronary angioplasty method is in any way manually performed and largely depends on the skillfulness and sensitiveness of the operator performing this method.

The operator is obliged to follow complex procedures, also because of the need of checking the parameters connected with the balloon catheter inflation and the effect of the inflation on the stenosis to be enlarged.

Furthermore the use of the equipments heretofore described involves high expenses, and this is due to the fact that hygienical reasons impose the use of disposable implements that are afterwards thrown away.

Not only the syringe is to be thrown away, although it has specific features and consenquently high cost, but also the pressure gauge and any other implement that comes in contacts with the inflating fluid.

Another drawback that has been found is the fact that the operator performing this method has also the task of compensating pressure leakages that occur in the equipment, and this must be done by continuously checking the pressure value shown by the gauge, or by the display, even when the equipment is temporarily made to pause.

The object of the present invention is to provide an apparatus that is able to automatically perform the balloon catheter method for coronary angioplasty, that allows a continuous check of the operative parameters, and that is designed in such a manner so as to considerably reduce the cost necessary for performing this method.

Another object of the present invention is to provide an apparatus for performing coronary angioplasty that is obtained by means of a simple technical solution, safe and reliable in working, and that is versatile to be used.

The above objects are achieved by means of an apparatus for performing the coronary angioplasty therapeutical method substantially made as described in Claim 1.

The apparatus described herein is able to automatically perform the inflation of the coronary angioplasty balloon catheter, in the meantime allowing the operator to constantly check the operative parameters.

The subject apparatus brings to a large decreasing of the cost for this therapeutical method.

The apparatus requires to replace only a disposable syringe that has a very low cost, and the catheter connected to it, instead of the complete equipment as requested when conventional apparatuses are used.

This is made possible because the other parts of the apparatus do not come in contact with the liquid forced into the catheter, particularly forcing and counting or metering means.

The replacement of the syringe is accomplished in a very simple and quick way, since the syringe is lodged inside a case that is situated outside the shell covering the apparatus and that is easily removable therefrom by unscrewing a blocking ring only.

The apparatus is always sterile because no parts of it come in contact with the patient, while the syringe and the catheter are replacd each time.

The characteristics of the invention that are not set forth in what is described above, will become apparent from the following description, taken in conjunction with the accompanying drawings in which:

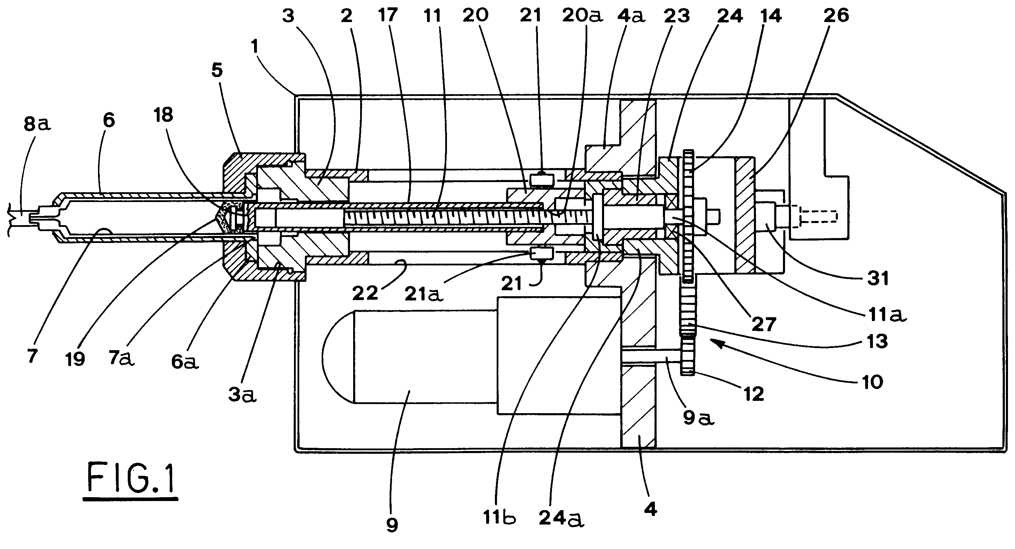

- Fig. 1 is a longitudinal sectional view of the apparatus for performing the coronary angioplasty balloon inflation;

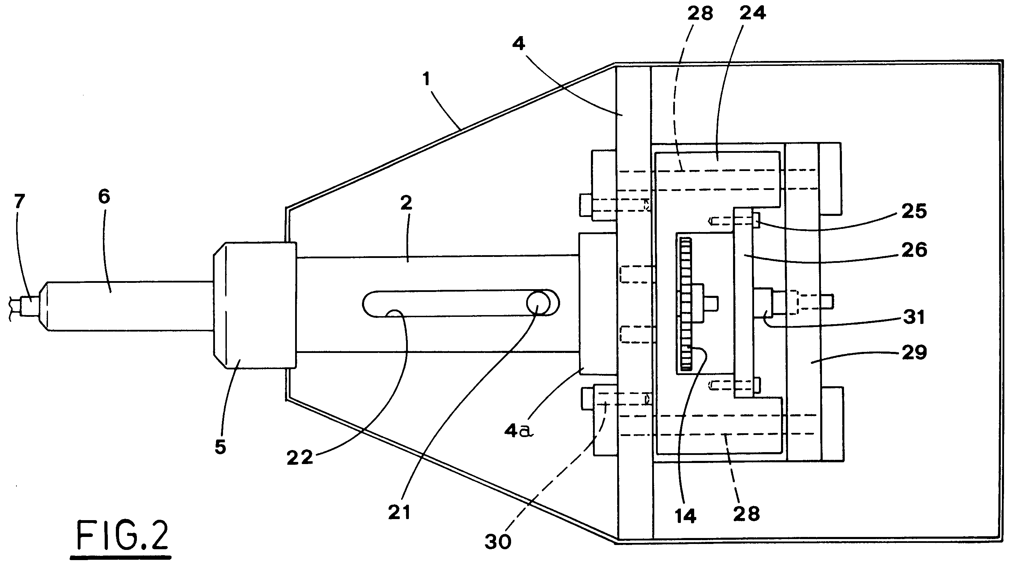

- Fig. 2 is a cross sectional view of the apparatus, taken along a horizontal plane;

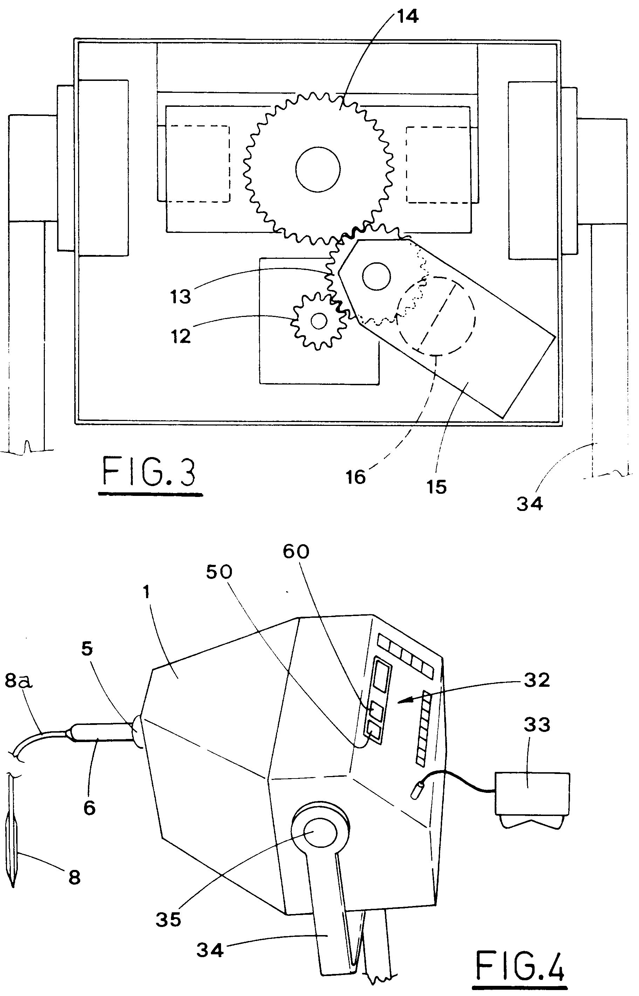

- Fig. 3 is another cross sectional view taken along a vertical plane;
- Fig. 4 is an external perspective view of the apparatus that is the subject matter.

Having reference to the figures cited above, the apparatus for inflating the balloon catheter 8 while performing the coronary angioplasty includes, inside a shell 1, a tubular body 2.

A head 3 is fastened to an extremity of the tubular body 2, while the remainder extremity of the tubular body 2 is inserted in a annular crown 4a made on a plate 4 located inside the same shell.

The head 3, that has a tubular shape, passes through a wall of the shell 1 and has a threadened portion 3a, located outside of the shell, onto which a ring 5 can be screwed for blocking a cylindrical case 6.

The blocking ring 5 clamps a flange 6a of the case 6 against the head 3.

The head 3, the threaded section 3a, the tubular case 6, and the blocking ring 5 form fixing means for a syringe 7.

The case 6 is designed for housing a conventional disposable type syringe 7, of very low cost.

The thickness of the case wall is such that a conventional disposable syringe can be used even if the inflation liquid is subjected to high pressure.

The syringe has a lip 7a, diametrically enlarged, that matches with a seat made in the case 6.

A catheter 8a, visible in part only in the figure, is connected to the syringe 7, this catheter being designed for performing the coronary angioplasty in accordance with known methods.
An electric motor means 9, with axle parallel to the tubular body, is also fastened to the plate 4.

A threaded rod 11, coaxially mounted inside the tubular body 2, is made to rotate by the motor 9 via a toothed drive link 10 that has the task of reducing the rotational speed transmitted from the motor 9 to the threaded rod 11.

The toothe drive link includes a pinion 12 that is keyed on the end of the shaft 9a of the motor 9, a gearwheel 14 fixed to the end of the shank 11a of the threaded rod 11, and a toothed wheel 13 that engages with both the pinion and the gearwheel 14 for transmitting the motion from the former to the latter.

The transmission toothed wheel 13 is rotatably supported by a small plate 15, and is designed to actuate a counter device 16, or encoder, mounted on the same small plate 15.

The threaded rod 11 runs through a sleeve 17 that passes through the head 3 so as to contact the plunger 18 of the syringe 7 and to push on it.

The plunger has a proper seal 19 so that the tightness between the plunger and the inner wall of the syringe is obtained.

Alternatively, the plunger 8 can be brought to push on a seal that is part of the disposable syringe.

A slide 20 that is slidably positioned inside the tubular body 2 and has a seat into which an end of the sleeve 17 is inserted.

The slide 20 has a inner threaded section 20a that engages with the threaded rod 11.

A pair of runners 21 are connected to the slide 20, in positions diametrically opposed to each other, and run inside longitudinal slots 22 made on the tubular body in positions also opposed to each other.

According to an improved embodiment, the apparatus can include small rollers 21a, that idle on the runners 21, as it is illustratively shown without limiting the scope of the invention.

This improved embodiment gets a better motion of the slide 20 along the tubular body, with positive effects on the overall working of the apparatus.

The threaded rod 11 features a shoulder 11b, by means of which it pushes on a saddle 24 that defines something like a bracket, with an axial bearing interposed between them (the bearing can be replaced with a simple bushing).

This bracket has a housing into which there is inserted the toothed wheel 14.

A crosspiece 26 is fastened to the bracket by means of screws 25.
The saddle 24 extends towards the plate 4 thus defining a tube 24a that is inserted in the hole of the plate, in corrispondence with the crown 4a, so as to abut against the bearing 23.

The shank 11a of the rod 11 is rotatably supported inside the tube 24a by means of a bearing 27.

The saddle 24 is a slidably mounted, with a short stroke, on a pair of stems 28 that project from the plate 4, with their axis parallel to the tubular body.

The stems 28 are integral parts of a frame 29 that is fastened to the plate 4 by means of screws 30.

The frame 29 has the task of holding a pressure sensor means 31, such as a load sensing cell.

The load cell 31 is positioned coaxially in respect of the tubular body 2, so that the crosspiece 26 of the saddle 24 touches the load cell and pushes on it.

The pressure value sensed by the load cell 31 is then shown on the display 32 that is located outside the shell 1, in a place where proper control and adjusment buttons are also located along with check pilot lights of the apparatus.

A double switch 33 is connected to the apparatus via a flexible cable, and is designed for controlling the rotational speed and direction of the electric motor 9, for inflating and deflating the balloon catheter 8 while performing the angioplasty method.

The shell 1 is hinged to a support 34 by means of the hinges 35, so as to properly bend the shell depending on the phase of the procedure.

In particular the part of the apparatus bearing the syringe is turned upwards before the beginning of the procedure, so that the air inside the syringe can come out.

Then, after that the catheter has been fixed to the syringe and filled with liquid, the same part of the apparatus is turned downwards, so that the air still present in the syringe is not injected in the catheter.

When the coronary angioplasty is performed by means of the apparatus just described, the catheter 8a is first inserted through the femoral artery until the balloon reaches the zone where the stenosis is, in accordance with the known method.

Then the balloon is inflated by actuating the motor 9 that rotates the threaded rod 11.

Because of this rotation, the slide 20 is made to advance along with the sleeve 17, that is an integral part of it and that pushes on the plunger 18 of the syringe 7.

The fluid inside the syringe is in this way forced through the catheter 8a, thus causing the balloon 8 to expand.

The actuation of the motor 9 is manually controlled by means of the double switch 33, that allows the gradual adjustment of the advancement speed in the proper manner, as well as the inversion of the rotation when required.

It is in this way possible to dose with precision the amount of fluid let in the catheter and the pressure thereof.

It must be pointed out that this switch 33 allows a fine adjustment of the motor working, and in both phases of inflation and deflation of the balloon.

And this gives the operator a high sensitiveness for actuating the apparatus, obviously with many advantages therefrom.

The apparatus detects the value of the pressure and of the amount of liquid injected by means of the load sensing cell 31 and of the encoder 16 respectively, and then it shows these values on the display 32.

Particularly, the load cell 31 is subjected to the axial thrust of the saddle 24 that is in turn subjected to the reaction of the threaded rod 11.
The encoder detects the number of rounds of the threaded rod 11, from which it is possible to reckon univocally the amount of liquid forced into the catheter 8a.

Pulses output by the encoder, in accordance with the number of rounds of the threaded rod 11, are then sent to an electronic control unit. The processed data are then displayed by the display 32.

This control unit is properly designed so that it can automatically control the rotation speed of the motor in such a way that the pressure value is kept constant when the apparatus is made to pause, even if leakages or the like occur in the circuit.

The pressure and quantity parameters detected can be sent to a peripheral unit, such as a plotter, so that a continuous graphic elaboration is obtained, and a diagram is drafted that shows the progress of the pressure parameters as a function of the parameters corresponding to the amount of liquid forced into the catheter. This diagram has a basic importance for checking the process.

The operator can in this way follow the trend of these parameters by a real time processing of the data, thus intervening to change them if it is necessary.

There are also two chronometers 50 and 60 connected to the apparatus. The first chronometer is started at the beginning of the angioplasty method performance, and shows the global time elapsed since the beginning.

The second chronometer is instead started each time that the apparatus is made to pause while the liquid inside the catheter must be kept at a constant pressure.

It is obviously possible that the apparatus be connected with a central processing unit, so that the parameters are stored for purpose of further check or statistical analysis and the like.

In the embodiment herein described and shown only as an example, the motion transmission between the motor and the rod 11 is obtained by means of gears.

The motion transmission can alternatively be obtained by means of other methods or systems, e.g. by means of a toothed belt that engages with two toothed wheels keyed on the motor shaft and on the shank of the threaded rod respectively.

The encoder is properly located so that it always detects the number of rounds of the threaded rod 11.

Also the saddle can be supported in a different manner, for example by means of a single bushing that is fixed to the plate 4, with a roller bearing placed therebetween.

## Claims

1. Apparatus comprising a disposable syringe being connected to a catheter (8a) for coronary angioplasty, and provided with sensors for performing the inflation of the balloon catheter used while performing the coronary angioplasty, **characterized in that** it includes:
a shell (1), for covering the apparatus;
a tubular body (2), located inside said shell (1);
said disposable syringe (7), located outside of the said shell, said syringe being fixed to an extremity of said tubular body, by fixing means passing through said shell (1);
a slide (20) that is located inside said tubular body (2) and axially slidable therein;
a sleeve (17) coaxially fastened to said slide (20), said sleeve (17) being designed to push onto the plunger (18) of said syringe (7), so as to force inflation fluid into said catheter (8a);
rotation preventing means co-operating with said tubular body (2) for preventing said slide (20) from axially rotating;
a threaded rod (11) coaxially running through said sleeve (17) and engaging with a threaded section (20a) of said slide (20), said threaded rod (11) being rotated by electric motor means (9) through a drive link (10);
a saddle (24), axially pushed by said threaded rod (11) and acting in turn on pressure sensing means (31).

2. Apparatus as in claim 1, **characterized in that** said drive link (10) includes a pinion (12) that is keyed on the shaft of the motor means (9), a gearwheel (14) fixed to the threaded rod (11), and a toothed wheel (13) that engages with both the pinion and the gearwheel (14) for transmitting the motion from the former to the latter.

3. Apparatus as in claim 1, **characterized in that** said drive link includes a toothed belt that links two toothed wheels keyed on the shaft of said electric motor means (9) and on said threaded rod (11) respectively.

4. Apparatus as in claim 2, **characterized in that** said drive link operates a counter device, or encoder, to meter the amount of liquid forced into the catheter (8a) by said syringe (7).

5. Apparatus as in claim 1, **characterized in that** said fixing means include:
a tube shaped head (3) that is fixed to an extremity of said tubular body (2) and that has a threaded section (3a);
a tubular case (6), into which the said syringe (7) is removably lodged;
a blocking ring (5) that engages with said threaded section (3a) of said head (3) for fixing said case (6) thereto.

6. Apparatus as in claim 1, **characterized in that** said rotation preventing means for said slide (20) include a pair of runners (21) running inside and along longitudinal slots made in said tubular body in positions that are opposed to each other.

7. Apparatus as in claim 6, **characterized in that** each runner (21) has mounted on it an idling roller (21a).

8. Apparatus as in claim 1, **characterized in that** said pressure sensing means include a load sensing cell axially positioned in respect of the said tubular body (2), said load cell being carried by a fixed frame (29) so that said saddle (24) acts on said load cell, with said saddle being slidably mounted on two stems (28) that are parallel to the said tubular body.

9. Apparatus as in claim 1, **characterized in that** said saddle (24) defines a bracket in which there is a housing into which there is inserted a toothed wheel (14) of said drive link (10), the bottom of said bracket having a hole for the passing of the shank (11a) of said threaded rod (11), the shank bearing said toothed wheel (14) and pushing onto said saddle (24) through an axial bearing (23).

10. Apparatus as in claim 1, **characterized in that** said saddle is supported by a single bushing fixed to said plate (4) with a roller axial bearing positioned therebetween.

## Patentansprüche

1. Ein Apparat, der eine Wegwerfspritze umfaßt, die mit einem Katheter (8a) für Koronar-Angio-Plastik verbunden ist, sowie Sensoren zum Aufblasen des Ballonkatheters, der während der Durchführung der Koronar-Gefäß-Plastik benutzt wird, dadurch gekennzeichnet, daß er weiterhin folgendes umfaßt:
ein Gehäuse (1) zum Abdecken des Apparates;
einen röhrenförmigen Körper (2), der sich innerhalb des genannten Gehäuses (1) befindet;
die genannte Wegwerfspritze (7), die sich außerhalb des genannten Gehäuses befindet und an dem äußeren Ende des genannten röhrenförmigen Körpers durch Befestigungselemente befestigt ist, die durch das genannte Gehäuse (1) führen;
einem Schlitten (20), der sich innerhalb des genannten röhrenförmigen Körpers (2) befindet und darin axial verschiebbar ist;
eine Büchse (17), die koachsial an dem genannten Schlitten (20) befestigt ist, wobei die genannte Büchse (17) so ausgelegt ist, daß sie auf den Kolben (18) der genannten Spritze (7) drückt, wodurch die Füllflüssigkeit in den genannten Katheter (8a) gedrückt wird;
Verdrehsicherungselemente, die durch Zusammenwirken mit dem röhrenförmigen Körper (2) verhindern, daß sich der genannte Schlitten (20) in Achsenrichtung dreht;
eine Gewindestange (11), die koachsial durch die genannte Büchse (20) verläuft und in einen Gewindebereich (20a) des genannten Schlittens (20) greift, wobei die Gewindestange (11) von elektrischen Antriebsmitteln (9) durch Übertragungsmittel (10) angetrieben wird;
einen Sattel (24), der von der genannten Gewindestange (11) in Achsenrichtung geschoben wird und wiederum auf Drucksensoren (31) wirkt.

2. Apparat wie in Patentanspruch 1, **dadurch gekennzeichnet daß** die genannten Übertragungsmittel (10) ein Ritzel (12) umfassen, das auf der Welle des Motors (9) verkeilt ist, sowie ein Getrieberad (14), das an der Gewindestange (11) angebracht ist, sowie ein Zahnrad (13), das sowohl in das Ritzel als auch in das Getrieberad (14) greift und die Kraft von dem ersten auf das letztere überträgt.

3. Apparat wie in Patentanspruch 1, **dadurch gekennzeichnet daß** die genannten Übertragungsmittel über einen Zahnriemen verfügen, der zwei Zahnräder auf der Welle der genannten elektrischen Antriebsmittel (9) bzw. auf der Gewindestange (11) miteinander verbindet.

4. Apparat wie in Patentanspruch 2, **dadurch gekennzeichnet daß** in den genannten Übertragungsmitteln eine Zählvorrichtung, oder ein Geber, arbeitet, der die Menge an Flüssigkeit mißt, die durch die genannte Spritze (7) in den Katheder (8a) gedrückt wird.

5. Apparat wie in Patentanspruch 1, **dadurch gekennzeichnet daß** die genannten Befestigungselemente folgendes umfassen:
einen röhrenförmigen Kopf (3), der am äußeren Ende des genannten röhrenförmigen Körpers (2) befestigt ist und einen Gewindebereich (3a) aufweist;
ein röhrenförmiges Gehäuse (6), in dem die genannte Spritze (7) herausnehmbar untergebracht ist;
einen Spannring (5), der zur Befestigung des genannten Gehäuses (6) in den Gewindebereich (3a) des genannten Kopfes (3) greift.

6. Apparat wie in Patentanspruch 1, **dadurch gekennzeichnet daß** die Verdrehsicherungselemente des genannten Schlittens (20) ein Paar Läufer (21) aufweisen, die innerhalb und entlang Längsschlitzen in dem röhrenförmigen Körper ausgebildet sind und einander gegenüberliegen.

7. Apparat wie in Patentanspruch 6, **dadurch gekennzeichnet daß** auf jedem Läufer (21) eine Leerlaufrolle (21a) sitzt.

8. Apparat wie in Patentanspruch 1, **dadurch gekennzeichnet daß** die genannten Drucksensoren eine Kraftmeßdose aufweisen, die in Achsenrichtung zu dem genannten röhrenförmigen Körper (2) positioniert ist, wobei diese Kraftmeßdose von einem festen Rahmen (29) getragen wird, so daß der genannte Sattel (24) auf die genannte Kraftmeßdose wirkt, wobei der genannte Sattel verschiebbar auf zwei Stäben (28) angebracht ist, die parallel zu dem genannten röhrenförmigen Körper stehen.

9. Apparat wie in Patentanspruch 1, **dadurch gekennzeichnet daß** der genannte Sattel (24) eine Klammer bildet, in der sich eine Aufnahme befindet, in der ein Zahnrad (14) der genannten Übertragungsmittel (10) sitzt, wobei das untere Ende der genannten Klammer eine Öffnung aufweist, durch die der Schaft (11a) der genannten Gewindestange (11) führt, wobei der Schaft das Zahnrad (14) trägt und durch ein Axiallager (23) auf den genannten Sattel (24) drückt.

10. Apparat wie in Patentanspruch 1, **dadurch gekennzeichnet daß** der genannte Sattel auf einer einzigen Buchse gelagert ist, die an der genannten Platte (4) mit einem dazwischen angeordneten Axialrollenlager befestigt ist.

## Revendications

1. Un appareil comprenant une seringue jetable et doté de capteurs, destiné au gonflement du cathéter à ballon utilisé pour l'angioplastie coronarienne, caractérisé en ce qu'il comprend :
une coque (1) destinée à couvrir l'appareil ;
un corps tubulaire (2) logé à l'intérieur de ladite coque (1) ;
ladite seringue jetable (7), située à l'extérieur de ladite coque, ladite seringue étant fixée à l'une des extrémités dudit corps tubulaire par un système de fixation passant à travers ladite coque (1);
une coulisse (20) logée à l'intérieur dudit corps tubulaire (2) et y coulissant selon un mouvement axial ;
un fourreau (17) fixé dans l'axe de ladite coulisse (20) , ledit fourreau (17) étant étudié pour exercer une pression sur le piston (18) de ladite seringue (7) de façon à faire pénétrer le fluide de gonflement dans ledit cathéter (8a) ;
un disposition anti-rotation fonctionnant de façon solidaire avec ledit corps tubulaire (2) afin d'empêcher ladite coulisse (20) de tourner de façon axiale ;
une tige filetée (11) se déplaçant selon un mouvement coaxial dans ledit fourreau (17) et s'engageant dans une section filetée (20a) de ladite coulisse (20), ladite tige filetée (11) étant mine selon un mouvement rotatif par un moteur électrique (9) au moyen d'un organe de transmission (10) ;
une selle (24), poussée selon un mouvement axial par ladite tige filetée (11) et agissant à son tour sur un dispositif de mesure de la pression (31).

2. Un appareil tel qu'à la revendication 1, caractérisé en ce que ledit organe de transmission (10) comprend un pignon (12) fixé à l'arbre du moteur (9), une roue à pignons (14) fixée à la tige filetée (11), et une roue dentée (13) s'engageant avec le pignon et la roue à pignons (14) pour transmettre le mouvement de l'un à l'autre.

3. Un appareil tel qu'à la revendication 1, caractérisé en ce que ledit organe de transmission comprend une courroie crantée reliant entre elles deux roues dentées fixées respectivement sur l'arbre dudit moteur électrique (9) et sur ladite tige filetée (11).

4. Un appareil tel qu'à la revendication 2, caractérisé en ce que ledit organe de transmission commande un dispositif de comptage, ou encodeur, qui mesure la quantité de liquide introduite dans le cathéter (8a) par ladite seringue (7).

5. Un appareil tel qu'à la revendication 1, caractérisé en ce que ledit système de fixation comprend :
une tête en forme de tube (3) fixée à l'une des extrémités dudit corps tubulaire (2) et présentant une section filetée (3a) ;
un logement tubulaire (6) dans lequel ladite seringue (7) est logée de façon amovible ;
une bague de blocage (5) s'engageant dans ladite section filetée (3a) de ladite tête (3) pour y fixer ledit logement (6).

6. Un appareil tel qu'à la revendication 1, caractérisé en ce que ledit dispositif anti-rotation pour ladite coulisse (20) comprend une paire de curseurs (21) se déplaçant à l'intérieur et le long de cannelures longitudinales pratiquées dans ledit corps tubulaire dans des positions opposées l'une à l'autre.

7. Un appareil tel qu'à la revendication 6, caractérisé en ce que chaque curseur (21) est doté d'un rouleau libre (21a).

8. Un appareil tel qu'à la revendication 1, caractérisé en ce que ledit dispositif de mesure de la pression comprend une cellule de mesure de charge montée dans l'axe du corps tubulaire (2), ladite cellule de mesure de charge étant supportée par un châssis fixe (29), de façon que ladite selle (24) agisse sur ladite cellule, ladite selle coulissant sur deux tiges (28) parallèles audit corps tubulaire.

9. Un appareil tel qu'à la revendication 1, caractérisé en ce que ladite selle (24) constitue un support dans lequel se trouve un logement dans lequel prend place une roue dentée (14) dudit organe de transmission (10), la base dudit support présentant un orifice permettant le passage de la pointe (11a) de ladite tige filetée (11), ladite pointe supportant ladite roue dentée (14) et poussant ladite selle (24) par un palier axial (23).

10. Un appareil tel qu'à la revendication 1, caractérisé en ce que ladite selle est supportée par une bague fixée à ladite platine (4), un palier axial tournant étant interposé entre elles.
